Europäisches Patentamt

European Patent Office

Office européen des brevets

(11) Publication number: **0 073 105**
**B1**

## (12) EUROPEAN PATENT SPECIFICATION

(45) Date of publication of patent specification: **25.09.85**

(21) Application number: **82304016.7**

(22) Date of filing: **29.07.82**

(51) Int. Cl.⁴: **C 07 C 85/11, C 07 C 87/22**

(54) Hydrogenation process.

(30) Priority: **14.08.81 US 293010**

(43) Date of publication of application:
**02.03.83 Bulletin 83/09**

(45) Publication of the grant of the patent:
**25.09.85 Bulletin 85/39**

(84) Designated Contracting States:
**DE FR GB IT**

(56) References cited:
**US-A-4 169 853**

**CHEMICAL ABSTRACTS, vol. 66, 1967, no.
75497p, page 7063, Columbus Ohio (USA); N.P.
SOKOLOVA et al.: "Catalytic reduction of
chloronitrobenzenes to chloroanilines. II. Use
of ruthenium in the reduction of p-
chloronitrobenzene"**

**The file contains technical information
submitted after the application was filed and
not included in this specification**

(73) Proprietor: **E.I. DU PONT DE NEMOURS AND
COMPANY
1007 Market Street
Wilmington Delaware 19898 (US)**

(72) Inventor: **Kosak, John Richard
103 Willow Spring Road Old Hobson Farm
Greenville Wilmington Delaware 19807 (US)**

(74) Representative: **Woodcraft, David Charles et al
BROOKES & MARTIN High Holborn House 52/54
High Holborn
London, WC1V 6SE (GB)**

(56) References cited:
**CHEMICAL ABSTRACTS, vol. 73, 1970, 34930a,
Columbus Ohio (USA); I.I. BAT et al.:
"Reduction of chloro- and
dichloronitrobenzenes on platinum Group
(rhodium, ruthenium, osmium) and rhenium
catalysts"
CHEMICAL ABSTRACTS, vol. 59, no. 4, 19th
August 1963, 3737c-e, Columbus Ohio (USA);
KAZUO TAYA: "Hydrogenation of aromatic
nitro compounds by ruthenium catalyst"**

Courier Press, Leamington Spa, England.

# 0 073 105

**Description**

The present invention relates to a process of reducing chloronitrobenzenes to the corresponding chloroaniline.

Russian Patent No. 188,982 and "Catalytic Reduction of Chloronitrobenzenes to Chloroanilines" by N. P. Sokolova et al. N. D. Zelinskii Institute of Organic Chemistry, Academy of Sciences of the USSR, pp 1830—1833 (1966) translated from Izvestiya Akademii Nauk SSSR, Seriya Khimicheskaya, No. 11, pp 1891—1895, November 1966, disclose the use of ruthenium supported on barium sulfate as the catalyst for reducing chloronitrobenzene to chloroaniline at pressures of 50 atm. and 100 atm. and temperatures up to 90°C.

British Patent Specification No. 1,064,959 discloses the hydrogenation of halonitrobenzene to haloaniline using a sulfide of platinum, rhodium, ruthenium or platinum as the catalyst. The use of platinum metal catalyst as the control runs is also disclosed.

U.S. Patent 4,164,481 discloses the regeneration of noble metal catalysts which have been used to hydrogenate nitrobenzene to aniline.

U.S. Patent 4,059,627 discloses the hydrogenation of chloronitrobenzene to chloroaniline in the presence of a sulfur compound such as a thioether.

U.S. Patent 4,169,853 discloses the use of solubilized ruthenium-containing complexes in combination with quaternary ammonium hydroxides to effect the reduction of various ortho-substituted nitroaromatics.

The present invention relates to an improved process for the reduction of chloronitrobenzenes to the corresponding chloroanilines. The improvement resides in the use of a supported catalyst which contains both ruthenium and platinum. Surprisingly, the use of this mixed catalyst provides the high selectivity associated with the use of ruthenium along with the rapid hydrogenation associated with the use of platinum.

In accordance with the present invention, reduction of a chloronitrobenzene to its corresponding chloroaniline is accomplished by contacting the chloronitrobenzene with hydrogen at a temperature of from 70°C to 160°C and a pressure of 200 to 800 psi: (i.e. 1380 kPag—5520 kPag) in the presence of a catalytic amount of a platinum group metal catalyst which is supported metallic ruthenium and supported metallic platinum in a weight ratio of ruthenium to platinum of 75:1 to 8:1. Preferably, the reaction is performed in the liquid phase and to this end temperature and pressure which admit of a liquid phase are preferably used. The catalyst generally amounts to 0:1 to 5% by weight based on the chloronitrobenzene starting material.

Further, it is preferred to perform the reaction at a temperature which not only permits a liquid phase reaction, but also which favors most efficient conversion with respect to rate of reaction, byproduct formation and minimum dechlorination. In this regard, temperatures from about 70°C to 160°C are operable, with 110°C to 150°C constituting the best range. At temperatures above about 160°C, the desired conversion takes place, but the product is contaminated with byproducts. Below 70°C, the reaction rate is such that undesirably prolonged reaction periods are required in order to obtain an economical degree of conversion.

The preferred range of pressure is 400 to 600 psi (i.e. 2760—4140 kPag). Above about 800 psi, the expense of creating the pressure becomes excessive with little gain in reactivity. Below about 200 psi, the reaction rate becomes excessively slow.

The catalyst will generally contain from 0.5 to 10 wt. % ruthenium as based on the total amount of catalyst and catalyst support present. Generally, the ratio of ruthenium to platinum present in the catalyst will vary from 75:1 to 8:1, with from 60:1 to 30:1 being the preferred range. The ruthenium and platinum may be present on the same catalyst support or the two metals may be separately supported and the two catalysts co-mixed in the reactor. The ruthenium and platinum catalyst may be prepared in various ways in order that a large surface area of metallic ruthenium or platinum per unit weight thereof is provided. A typical preparation includes depositing metallic ruthenium or platinum, for example by spraying, saturating, or precipitating a salt followed by reduction or the like, on a finely divided inert support such as activated alumina or activated carbon. Generally, the support will have a particle size of less than 200 mesh. Other suitable supports include finely divided silicon dioxide, carbon black, alumina, calcium silicate, sodium silicate, mixed silicates such as calcium-aluminium silicate and calcium-sodium silicate, talc, calcium carbonate, and clay.

Generally, the reaction time will be from 20 to 300 minutes. The reaction may be run in either the batch or continuous mode.

While the process of the present invention is generally applicable to any chloronitrobenzene, the chloronitrobenzens containing one to three chlorine atoms and one nitro group are preferred. The preferred chloronitrobenzens are 1-nitro-3,4-dichlorobenzene, 1-nitro-2-chlorobenzene, 1-nitro-4-chlorobenzene, 1-nitro-2,5-dichlorobenzene and 1-nitro-2,3-dichlorobenezene.

The process of the present invention minimizes production of the chloroazobenzene corresponding to the nitrochlorobenzene starting material. 3,3',4,4'-Tetrachloroazobenzene is an acnegen, and, therefore, its production is undesirable.

The chloroanilines produced by the process of the present invention are useful as intermediates in the production of herbicides, dyes and pigments.

2

The following specific Examples are intended to illustrate the invention. In each of Examples 1—12 as reported in Table I, a 1 litre titanium autoclave is charged with 300 g of nitrodichlorobenzene containing 86.3% of the 1 nitro-3,4-dichlorobenzene isomer and 1.2 g of a commercial 5 wt.% ruthenium on finely divided carbon catalyst. The platinum catalyst (Pt-Cat) used in Examples 1—10 is a commercial 5 wt.% platinum on finely divided carbon catalyst. The platinum catalyst used in Examples 11 and 12 contains 5 wt.% platinum, 1.25 wt.% nickel and 1.25 wt.% chromium supported on finely divided carbon, prepared by the process of U.S. Patent 3,546,297. In each of Examples 1—12, the autoclave is pressured to 500 psig (3450 kPag) and repressured to 500 psig (3450 kPag) whenever the pressure drops to 400 psig (2760 kPag). In control Example 1, a higher temperature of 140/150°C (145±5°C) is used which effectively doubles the reaction rate obtained when 130/140°C (135±5°C) is used. In Table I "DECHL" means dechlorination, "DCA" means the 3,4-dichloroaniline product and "TCAB" means 3,3'4,4'-tetrachloroazobenzene. The "DCA" yields reported in Table I are based on the 1-nitro-3,4-dichlorobenzene in the starting material.

## TABLE I

| Ex | Pt-cat g | Time min. | Temp °C | DECHL % | DCA % | TCAB PPM |
|---|---|---|---|---|---|---|
| 1 | 0 | 122 | 140/150 | 0.747 | 97.8 | — |
| 2 | 0.02 | 165 | 130/140 | 0.827 | 96.3 | — |
| 3 | 0.02 | 163 | 130/140 | 0.711 | 95.6 | — |
| 4 | 0.03 | 105 | 130/140 | 0.786 | 96.8 | 217 |
| 5 | 0.03 | 106 | 130/140 | 0.689 | 97.0 | 64 |
| 6 | 0.04 | 72 | 130/140 | 1.051 | 96.0 | 15 |
| 7 | 0.04 | 97 | 130/140 | 0.704 | 9.65 | 483 |
| 8 | 0.04 | 89 | 130/140 | 0.486 | 95.8 | 96 |
| 9 | 0.05 | 56 | 130/140 | 0.742 | 95.5 | 2830 |
| 10 | 0.05 | 58 | 130/140 | 1.056 | 96.3 | 2953 |
| 11 | 0.1 | 67 | 130/140 | 0.405 | 97.8 | 1346 |
| 12 | 0.1 | 66 | 130/140 | 0.383 | 98.0 | 2725 |

**Claims**

1. A process of reducing a chloronitrobenzene to the corresponding chloroaniline which process comprises contacting said chloronitrobenzene with hydrogen at a temperature of from 70°C to 160°C and a pressure of from 200 to 800 psi (1380 to 5520 kPag) in the presence of a catalytic amount of a platinum group metal catalyst, characterised in that, as the platinum group metal catalyst, supported metallic ruthenium and supported metallic platinum are used in a weight ratio of ruthenium to platinum of from 75:1 to 8:1.

2. A process as claimed in Claim 1 wherein the catalyst is supported on a finely divided inert carrier, said ruthenium comprising from 0.5 to 10 weight percent of the carrier.

3. A process as claimed in Claim 1 or Claim 2 wherein the catalyst including ruthenium, platinum and support is present in an amount of from 0.1 to 5 percent by weight based on the chloronitrobenzene starting material.

4. A process as claimed in any preceding claim wherein the chloronitrobenzene starting material contains from 1 to 3 chlorine atoms and one nitro group.

5. A process as claimed in any preceding claim wherein the contact is effected at a temperature of from 110° to 150°C.

6. A process as claimed in any preceding claim wherein the contact is effected at a pressure from 400 to 600 psi (2760 to 4140 kPag)

7. A process as claimed in any preceding claim wherein the chloronitrobenzene starting material is 1-nitro-3,4-dichlorobenzene, 1-nitro-2-chlorobenzene, 1-nitro-4-chlorobenzene, 1-nitro-2,5-dichlorobenzene or 1-nitro-2,3-dichlorobenzene.

8. A process as claimed in any preceding claim wherein the catalyst support is carbon black or alumina.

9. A process as claimed in any preceding claim wherein the weight ratio of ruthenium to platinum is from 60:1 to 30:1.

# 0 073 105

**Patentansprüche**

1. Verfahren zur Reduktion eines Chlornitrobenzols zum entsprechenden Chloranilin, wobei das Chlornitrobenzol mit Wasserstoff bei einer Temperatur von 70°C bis 160°C und einem Druck von 200 bis 800 psi (1380 bis 5520 kPag) in Gegenwart einer katalytischen Menge eines Katalysators aus der Platinmetallgruppe in Berührung gebracht wird, dadurch gekennzeichnet, daß als Katalysator aus der Platinmetallgruppe mit Trägerstoff versehenes metallisches Ruthenium und mit Trägerstoff versehenes metallisches Platin in einem Gewichtsverhältnis von Ruthenium zu Platin von 75:1 bis 8:1 verwendet werden.

2. Verfahren nach Anspruch 1, worin der Katalysator auf einem feinverteilten inerten Träger angeordnet ist, wobei das Ruthenium 0,5 bis 10 Gew.-% des Trägers umfaßt.

3. Verfahren nach Anspruch 1 oder Anspruch 2, worin der Katalysator einschließlich Ruthenium, Platin und Träger in einer Menge von 0,1 bis 5 Gew.-% bezogen auf das Chlornitrobenzol-Ausgangsmaterial vorhanden ist.

4. Verfahren nach einem der voranstehenden Ansprüche, worin das Chlornitrobenzol-Ausgangsmaterial 1 bis 3 Chloratome und eine Nitrogruppe enthält.

5. Verfahren nach einem der voranstehenden Ansprüche, worin die Berührung bei einer Temperatur von 110° bis 150°C bewirkt wird.

6. Verfahren nach einem der voranstehenden Ansprüche, worin die Berührung bei einem Druck von 400 bis 600 psi (2760 bis 4140 kPag) bewirkt wird.

7. Verfahren nach einem der voranstehenden Ansprüche, worin das Chlornitrobenzol-Ausgangsmaterial das 1-Nitro-3,4-dichlorbenzol, 1-Nitro-2-chlorbenzol, 1-Nitro-4-chlorbenzol, 1-Nitro-2,5-dichlorbenzol oder 1-Nitro-2,3-dichlorbenzol ist.

8. Verfahren nach einem der voranstehenden Ansprüche, worin der Katalysator-Träger Ruß oder Aluminiumoxid ist.

9. Verfahren nach einem der voranstehenden Ansprüche, worin das Gewichtsverhältnis von Ruthenium zu Platin bei 60:1 bis 30:1 liegt.

**Revendications**

1. Procédé de réduction d'un chloronitrobenzène en la chloroaniline correspondante, qui comprend la mise en contact dudit chloronitrobenzène avec de l'hydrogène à une température de 70 à 160°C et à une pression relative de 1380 à 5520 kPa en présence d'une quantité catalytique d'un catalyseur métallique du groupe du platine, caractérisé en ce que, en tant que catalyseur métallique du groupe du platine, il est fait appel à du ruthénium métallique sur support et à du platine métallique sur support avec un rapport pondéral du ruthénium au platine de 75/1 à 8/1.

2. Procédé selon la revendication 1 dans lequel le catalyseur est supporté par un support inerte finement divisé, ledit ruthénium constituant de 0,5 à 10 pour cent en poids du support.

3. Procédé selon la revendication 1 ou la revendication 2 dans lequel le catalyseur, ruthénium, platine et support compris, est présent en une proportion de 0,1 à 5 pour cent en poids par rapport au chloronitrobenzène de départ.

4. Procédé selon l'une quelconque des revendications précédentes dans lequel le chloronitrobenzène de départ contient de 1 à 3 atomes de chlore et un groupe nitro.

5. Procédé selon l'une quelconque des revendications précédentes dans lequel la mise en contact est effectuée à une température de 110 à 150°C.

6. Procédé selon l'une quelconque des revendications précédentes dans lequel la mise en contact est effectuée à une pression relative de 2760 à 4140 kPa.

7. Procédé selon l'une quelconque des revendications précédentes dans lequel le chloronitrobenzène de départ est du 1-nitro-3,4-dichlorobenzène, du 1-nitro-2-chlorobenzène, du 1-nitro-4-chlorobenzène, du 1-nitro-2,5-dichlorobenzène ou du 1-nitro-2,3-dichlorobenzène.

8. Procédé selon l'une quelconque des revendications précédentes dans lequel le support du catalyseur est du noir de carbone ou de l'alumine.

9. Procédé selon l'une quelconque des revendications précédentes dans lequel le rapport pondéral du ruthénium au platine est de 60/1 à 30/1.

4